# EUROPEAN PATENT APPLICATION

(11) **EP 4 764 492 A1**
(43) Date of publication of application: **24.06.2026**
(21) Application number: 25215186.5
(22) Date of filing: 12.11.2025
(51) Int. Cl.: G01N 33/00

(54) **AN INTEGRATED PUMP SYSTEM FOR A GAS DETECTOR AND A METHOD THEREOF**

(30) Priority: 17.12.2024 CN 202411863841
(71) Applicant: Life Safety Distribution GmbH, 1180 Rolle (CH)
(72) Inventor: HUANG, Chuang, Charlotte, 28202 (US); TAN, Xiufeng, Charlotte, 28202 (US); XU, Yuhui, Charlotte, 28202 (US); HU, Yu, Charlotte, 28202 (US)
(74) Representative: Haseltine Lake Kempner LLP

(57) **Abstract**

Systems, apparatuses, and method for gas detections are disclosed. An exemplary system is a device that includes gas detector and a pump housing coupled to the gas detector. The gas detector comprises a first plurality of terminal electrodes, a plurality of switches, and a first infrared (IR) sensor. The pump housing comprises a pump, a second plurality of terminal electrodes, a plurality of magnets, and a second infrared (IR) sensor. The second plurality of electrodes are configured to align with the first plurality of terminal electrodes. The plurality of magnets are configured to control the plurality of switches to enable or disable flow of electricity from the first plurality of terminal electrodes to the second plurality of terminal electrodes to operate the pump for detecting a presence of gas. The second IR sensor is configured to communicate with the first IR sensor of the gas detector.

## Description

### TECHNOLOGICAL FIELD

Example embodiments of the present disclosure generally relates to a gas detection system, and more particularly relates to a gas detection system with an integrated external pump.

### BACKGROUND

Gas detectors are available with either an internal pump or an external pump. The gas detectors equipped with the external pump face several operational issues such as high cost, driven by the need for a separate display and a long probe to connect the external pumps to the gas detectors. The separate display and the long probe add complexity and increase both production and maintenance costs. Additionally, the external pump often requires a separate battery source for power, which adds to the overall gas detectors burden. Operators must monitor power levels of both the gas detector and the external pump, leading to inefficiency and operational difficulties, particularly in environments where quick, reliable monitoring is crucial. Such complexity of managing the gas detectors, the external pumps, the separate display, and the long probe at once, alongside the frequent need for maintenance due to the long probe's wear and tear, further increases costs and decreases ease of use.

The inventors have identified numerous areas of improvement in the existing technologies and processes, which are the subjects of embodiments described herein. Through applied effort, ingenuity, and innovation, many of these deficiencies, challenges, and problems have been solved by developing solutions that are included in embodiments of the present disclosure, some examples of which are described in detail herein.

### BRIEF SUMMARY

The following presents a simplified summary in order to provide a basic understanding of some aspects of the present disclosure. This summary is not an extensive overview and is intended to neither identify key or critical elements nor delineate the scope of such elements. Its purpose is to present some concepts of the described features in a simplified form as a prelude to the more detailed description that is presented later.

In an example embodiment, a device is disclosed. The device comprises a gas detector. The gas detector comprises a first plurality of terminal electrodes, a plurality of switches, and a first infrared (IR) sensor. The device further comprises a pump housing coupled to the gas detector. The pump housing comprises a pump. The pump housing further comprises a second plurality of terminal electrodes configured to align with the first plurality of terminal electrodes. The pump housing further comprises a plurality of magnets configured to control the plurality of switches to enable or disable flow of electricity from the first plurality of terminal electrodes to the second plurality of terminal electrodes to operate the pump for detecting a presence of gas. The pump housing further comprises a second infrared (IR) sensor configured to communicate with the first IR sensor of the gas detector.

In some embodiments, the pump is configured to draw air via a gas input port of the pump housing and transfer the air to a plurality of gas sensors via a plurality of gas output ports to detect the presence of the gas.

In some embodiments, the pump housing further comprises a slot that defines a J-shaped profile for connecting the gas detector with the pump housing.

In some embodiments, the first plurality of terminal electrodes comprises a first power terminal electrode and a first ground terminal electrode and the second plurality of terminal electrodes comprise a second power terminal electrode and a second ground terminal electrode.

In some embodiments, the gas detector further comprises a battery management unit connected to the first power terminal electrode and a battery connected to the first plurality of terminal electrodes. The pump housing further comprises a power management unit and a plurality of gas output ports.

In some embodiments, the plurality of switches comprises a single pole double throw (SPDT) switch connected to the first power terminal electrode and a single pole single throw (SPST) switch connected to the battery.

In some embodiments, the SPDT switch and the SPST switch are configured to respond to a magnetic field of the plurality of magnets such that when the pump housing is coupled to the gas detector, the SPDT switch is in an open state and the SPST switch is in a closed state. The SPDT switch and the SPST switch are further configured to respond to a magnetic field of the plurality of magnets such that when the pump housing is not coupled to the gas detector, the SPDT switch is in a closed state and the SPST switch is in an open state.

In some embodiments, when the SPDT switch is in the open state and the SPST switch is in the closed state, the battery is configured to transmit power to the pump via the first power terminal electrode and the second power terminal electrode.

In some embodiments, when the SPDT switch is in the closed state and the SPST switch is in the open state, the battery management unit is configured to control supply of power received from an external power source to recharge the battery.

In some embodiments, the SPST switch is positioned at a different direction with the SPDT switch so that the SPST switch is configured to be sensitive to a different magnetic field direction than the SPDT switch.

In another example embodiment, a method is disclosed. The method comprising steps of coupling a pump housing to a gas detector. The gas detector comprises a first plurality of terminal electrodes, a plurality of switches, and a first infrared (IR) sensor. The pump housing comprises a pump. The pump housing further comprises a second plurality of terminal electrodes configured to align with the first plurality of terminal electrodes. The pump housing further comprises a plurality of magnets configured to control the plurality of switches to enable or disable flow of electricity from the first plurality of terminal electrodes to the second plurality of terminal electrodes to operate the pump for detecting a presence of gas. The pump housing further comprises a second infrared (IR) sensor configured to communicate with the first IR sensor of the gas detector.

In some embodiments, the method comprising steps of drawing, via the pump, air via a gas input port of the pump housing. The method further comprising steps of transferring the air to the plurality of gas sensors in the gas detector via a plurality of gas output ports. The method further comprising steps of detecting the presence of the gas with one or more of the plurality of gas sensors.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the present disclosure in any way. It will be appreciated that the scope of the present disclosure encompasses many potential embodiments in addition to those here summarized, some of which will be further described below.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 illustrates a circuit diagram of a device comprising a pump housing and a gas detector in accordance with an example embodiment of the present disclosure;
FIG. 2 illustrates a side view of the gas detector integrated into a slot of the pump housing in accordance with the example embodiment of the present disclosure;
FIG. 3A illustrates a front view of the pump housing in accordance with the example embodiment of the present disclosure;
FIG. 3B illustrates a side view of the pump housing comprising the slot in accordance with the example embodiment of the present disclosure;
FIG. 4A illustrates a front view of the gas detector in accordance with the example embodiment of the present disclosure;
FIG. 4B illustrates a side view of the gas detector in accordance with the example embodiment of the present disclosure; and
FIG. 5 illustrates a flowchart showing a method for detecting a presence of gas in accordance with the example embodiment of the present disclosure.

### DETAILED DESCRIPTION

Some embodiments will now be described more fully hereinafter with reference to the accompanying drawings, in which some, but not all, embodiments are shown. Indeed, various embodiments may be embodied in many different forms and should not be construed as limited to the embodiments set forth herein; rather, these embodiments are provided so that this disclosure will satisfy applicable legal requirements.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in dashed line for visibility of the underlying components.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in various embodiments," "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments or it may be excluded.

The present disclosure provides various embodiments of a system, apparatus, and method for gas detection. Embodiments may include a device with a gas detector and a pump housing. Embodiments may be configured to control a plurality of switches to enable or disable flow of electricity from a first plurality of terminal electrodes to a second plurality of terminal electrodes to operate a pump for detecting a presence of gas. Embodiments may be further configured to draw air via a gas input port of the pump housing and transfer the air to a plurality of gas sensors via the plurality of gas output ports to detect the presence of the gas. Embodiments may be further configured to respond to a magnetic field of a plurality of magnets. Embodiments may be configured to transmit power to the pump via a first power terminal electrode and a second power terminal electrode. Embodiments may be further configured to control supply of power received from an external power source to recharge a battery.

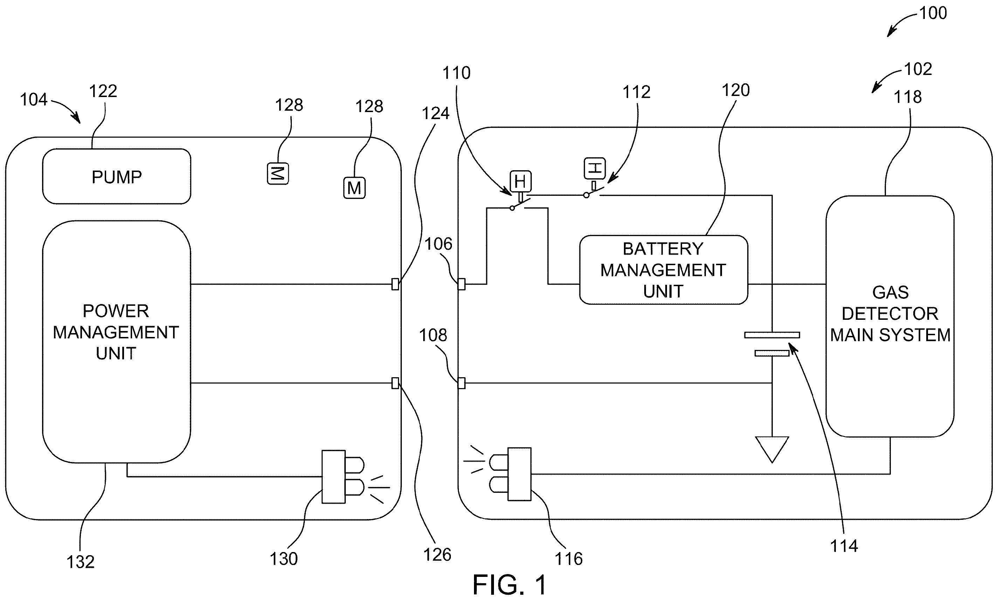

FIG. 1 illustrates a circuit diagram of a device 100 comprising a pump housing 104 and a gas detector 102, in accordance with an example embodiment of the present disclosure.

In some embodiments, the device 100 may comprise the gas detector 102 and the pump housing 104. In some embodiments, the gas detector 102 may be configured to detect a presence of a gas within a proximity of the gas detector 102. The gas detector 102 may ensure safety of personnel by alerting the personnel to potentially dangerous gas leaks or concentration of the gas. The gas may further correspond to toxic gases. The gas detector 102 may be widely used in industrial, commercial, and residential environments. The gas detector 102 may be configured to detect the presence of the gas and prevent accidents, health hazards, or explosions caused by the presence of the gas.

In some embodiments, the gas detector 102 may be configured to detect the gas. The gas may comprise, but is not limited to, carbon monoxide (CO), methane (CH₄), hydrogen sulfide (H₂S), and oxygen (O₂). Once a gas is detected, the gas detector 102 may measure concentration of the detected gas and may compare the measured concentration of the detected gas to a predefined threshold. If the measured gas concentration exceeds the predefined threshold, the gas detector 102 may raise an alert.

In some embodiments, the gas detector 102 may comprise a first plurality of terminal electrodes, a plurality of switches, and a first infrared (IR) sensor 116. The first plurality of terminal electrodes may comprise a first power terminal electrode 106 and a first ground terminal electrode 108. Further, the plurality of switches may comprise a single pole double throw (SPDT) switch 110 connected to the first power terminal electrode 106 and a single pole single throw (SPST) switch 112 connected to a battery 114.

In some embodiments, a gas detector main system 118 may comprise a plurality of gas sensors. The plurality of gas sensors may be configured to detect the presence of the gas or a combinations of gases. The combination of gases may include, but is not limited to CO, H₂S, and O₂. The plurality of gas sensors may produce a measurable electrical signal in response to the presence of the gas. In some embodiments, the gas detector 102 may comprise the battery 114 and a battery management unit 120. The battery 114 may correspond to a rechargeable battery. The battery management unit 120 may monitor battery levels, manages charging cycles, and optimizes power flow to the device 100.

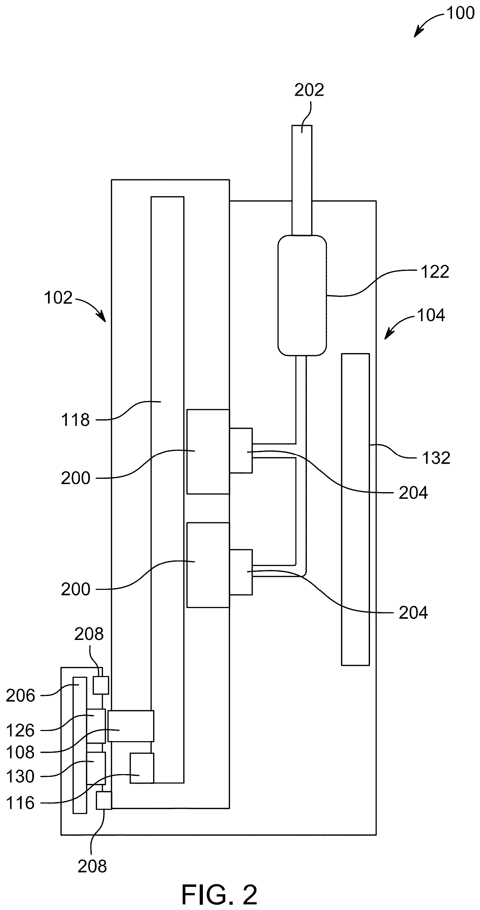

In some embodiments, the device 100 may comprise the pump housing 104. The pump housing 104 may comprise a pump 122, a second plurality of terminal electrodes, a plurality of magnets 128, and a second infrared (IR) sensor 130. The second plurality of terminal electrodes may comprise a second power terminal electrode 124 and a second ground terminal electrode 126. In some embodiments, the pump housing 104 may correspond to a pumping unit. The pump housing 104 may be configured to sample the gas from a proximity of the gas detector 102. The pump housing 104 may be further configured to continuously pull in the gas through an input port of the pump housing (as shown in FIG. 2).

In some embodiments, the second plurality of terminal electrodes may be configured to align with the first plurality of terminal electrodes. The second power terminal electrode 124 may be configured to align with the first power terminal electrode 106. Further, the second ground terminal electrode 126 may be configured to align with the first ground terminal electrode 108. The second power terminal electrode 124 may be configured to receive electrical energy from the gas detector 102. Further, the second ground terminal electrode 126 may align with the first ground terminal electrode 108 to complete a power circuit. In some embodiments, the second plurality of terminal electrodes may align with the first plurality of terminal electrodes when the pump housing 104 is physically attached to the gas detector 102.

In some embodiments, the pump housing 104 may further comprise a power management unit 132. The power management unit 132 may regulate power flow from the battery 114 of the gas detector 102, through the aligned first plurality of terminal electrodes, the second plurality of terminal electrodes and to the pump 122. The power management unit 132 may manage power distribution to ensure that the pump 122 may operate effectively without causing excessive drain on the battery 114. The power management unit 132 may be further configured to manage operation of the pump housing 104. The power management unit 132 may be further configured to manage power flow between the pump housing 104 and the gas detector 102.

In some embodiments, the power management unit 132 may be configured to distribute a desired amount of the power to the pump 122. The power management unit 132 may further ensure that the pump housing 104 may not draw too much power from the gas detector 102. The power management unit 132 may be configured to activate the pump 122 by allowing the gas detector 102 to supply the power to the pump housing 104.

In some embodiments, the plurality of magnets 128 may be configured to control the plurality of switches to enable or disable flow of electricity from the first plurality of terminal electrodes to the second plurality of terminal electrodes to operate the pump 122 for detecting the presence of the gas. Further, the second IR sensor 130 may be configured to communicate with the first IR sensor 116 of the gas detector 102. In some embodiments, the first plurality of terminal electrodes and the second plurality of terminal electrodes may be configured to exchange the power between the pump housing 104 and the gas detector 102.

In some embodiments, the SPDT switch 110 and the SPST switch 112 may be configured to respond to a magnetic field of the plurality of magnets 128 such that when the pump housing 104 is coupled to the gas detector 102, the SPDT switch 110 may be in an open state and the SPST switch 112 may be in a closed state. Further, when the SPDT switch 110 is in the open state and the SPST switch 112 is in the closed state, the battery 114 may be configured to transmit the power to the pump 122 via the first power terminal electrode 106 and the second power terminal electrode 124.

In some embodiments, the SPDT switch 110 and the SPST switch 112 may be configured to respond to a magnetic field of the plurality of magnets 128 such that when the pump housing 104 is not coupled to the gas detector 102, the SPDT switch 110 may be in a closed state and the SPST switch 112 may be in an open state. Further, when the SPDT switch 110 is in the closed state and the SPST switch 112 is in the open state, the battery management unit 120 may be configured to control supply of the power received from the external power source to recharge the battery 114.

In some embodiments, the open state and the closed state of the SPDT switch 110 and the SPST switch 112 may be controlled by the proximity or alignment of the pump housing 104 with the gas detector 102. When the pump housing 104 is properly attached to the gas detector 102, the magnetic field may act on the SPDT switch 110 and the SPST switch 112 to set the SPDT switch 110 and the SPST switch 112 in the respective states.

In some embodiments, the plurality of magnets 128 may be configured to align with the plurality of switches. The plurality of magnets 128 may comprise two magnets in one or more directions. Further, the plurality of switches may comprise two magnetic switches. The two magnetic switches may correspond to the SPDT switch 110 and the SPST switch 112. In some embodiments, the SPST switch 112 may be positioned at a different direction with the SPDT switch 110 so that the SPST switch 112 may be configured to be sensitive to a different magnetic field direction than the SPDT switch 110.

In some embodiments, the gas detector 102 may be configured to toggle between one or more operational modes within the gas detector 102. The one or more operational modes may comprise a charging mode and a backward power supply mode. In the charging mode, the first power terminal electrode 106 may be configured to charge the battery 114. In the backward power supply mode, the gas detector 102 may supply the power to the pump housing 104 through the first power terminal electrode 106. The SPDT switch 110 may be sensitive to a magnetic field's direction and may only trigger when the corresponding magnet from the plurality of magnets 128 positioned on the pump structure 102 is aligned in a desired direction. In some embodiments, when the pump housing 104 may be inserted within the gas detector 102, the SPDT switch 110 may detect the magnetic field of the corresponding magnet from the plurality of magnets 128 positioned on the pump housing 104 and may switch from the charging mode to the backward power supply mode. The SPDT switch 110 may be configured to allow the gas detector 102 to power the pump housing 104.

In some embodiments, The SPST switch 112 may be configured to prevent false triggering of the SPDT switch 110 by surrounding magnetic fields. The SPST switch 112 may be configured to ensure that the SPDT switch 110 may not accidentally toggle into a different operational mode from the one or more operational mode. In some embodiments, the SPST switch 112 may be placed in a different direction compared to the SPDT switch 110. The SPST switch 112 may be sensitive to a magnetic field different to the magnetic field's direction which is sensitive to the SPDT switch 110.

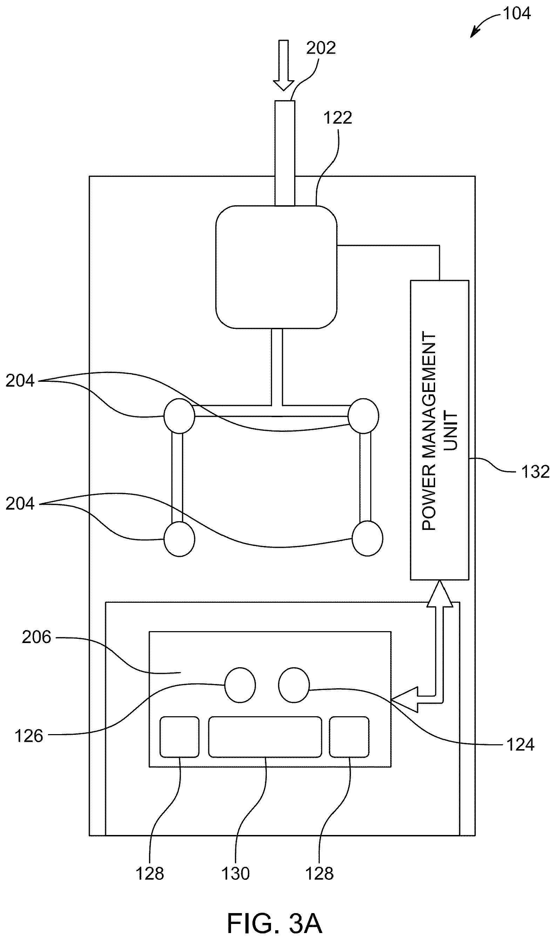

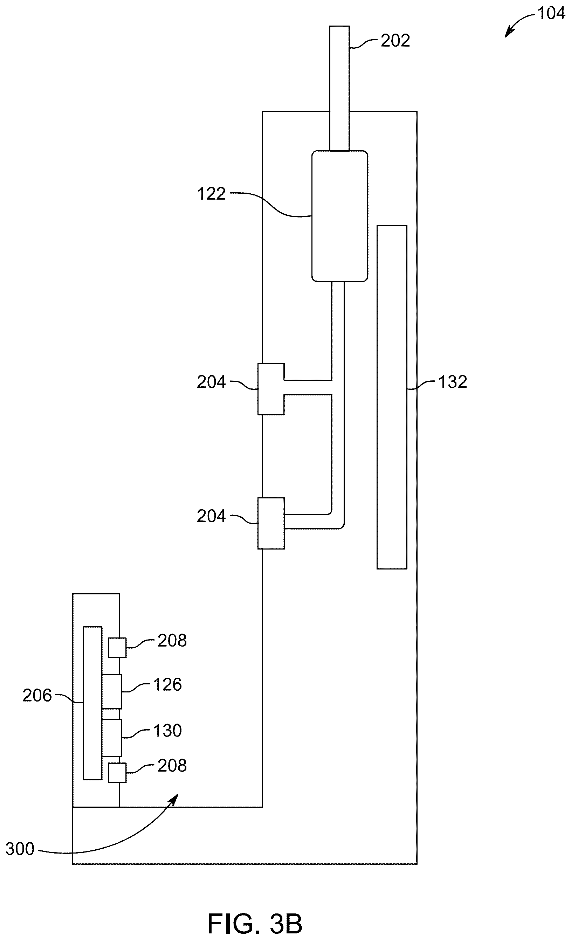

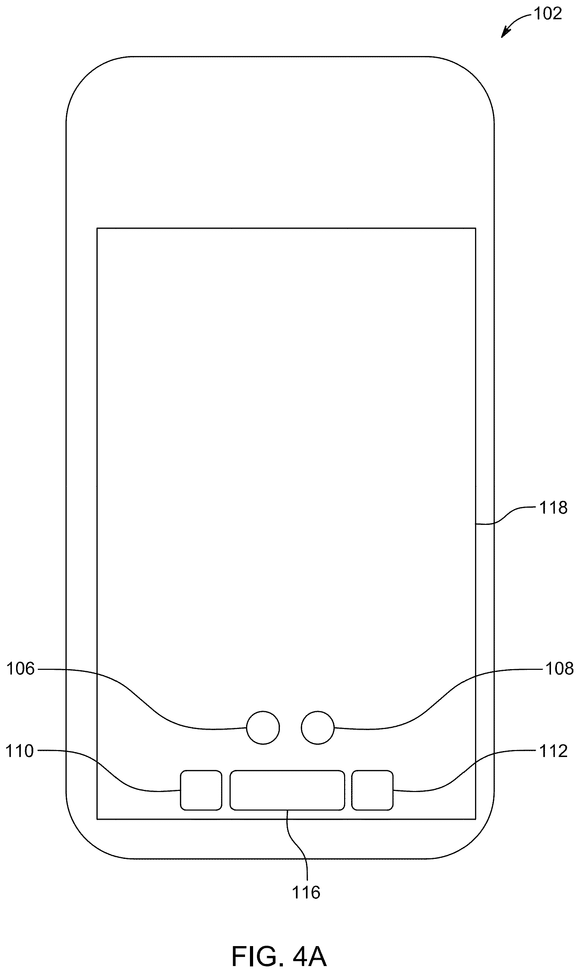

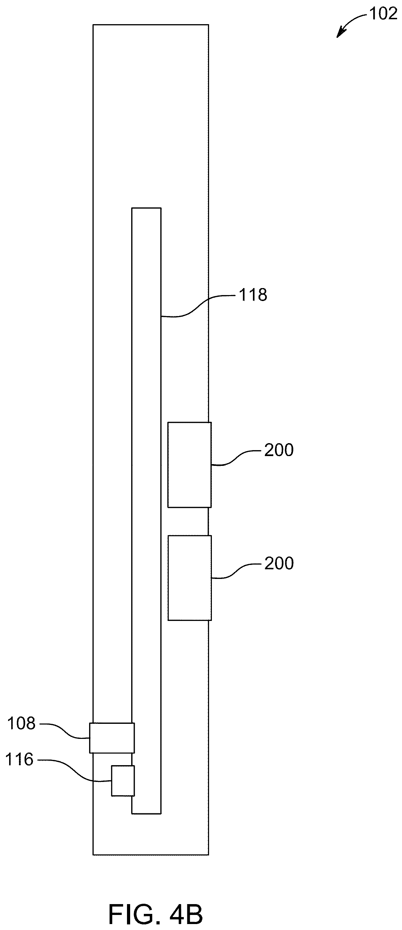

FIG. 2 illustrates a side view of the gas detector 102 integrated into a slot 300 of the pump housing 104, in accordance with the example embodiment of the present disclosure. FIG. 3A illustrates a front view of the pump housing 104, in accordance with the example embodiment of the present disclosure. FIG. 3B illustrates a side view of the pump housing 104 comprising the slot 300, in accordance with the example embodiment of the present disclosure. FIG. 4A illustrates a front view of the gas detector 102, in accordance with the example embodiment of the present disclosure. FIG. 4B illustrates a side view of the gas detector 102, in accordance with the example embodiment of the present disclosure.

In some embodiments, when the gas detector 102 is placed within the slot 300 of the pump housing 104, the gas detector 102 and the pump housing 104 may be connected with each other. Further, when the gas detector 102 is placed within the slot 300 of the pump housing 104, the first IR sensor 116 in the gas detector 102 may send one or more commands to the second IR sensor 130 in the pump housing 104. The one or more commands may allow the gas detector 102 to control the pump 122. In some embodiments, if the gas detector 102 is removed from the slot 300 of the pump housing 104, the first IR sensor 116 and the second IR sensor 130 may stop communicating with each other, and the gas detector 102 may automatically recognize change in configuration.

In some embodiments, the second IR sensor 130 in the pump housing 104 may establish a direct infrared communication link with the first IR sensor 116 positioned within the gas detector 102. The first IR sensor 116 and the second IR sensor 130 may be configured to allow the gas detector 102 and the pump housing 104 to communicate with each other wirelessly. The direct infrared communication link between the gas detector 102 and the pump housing 104 may enable the gas detector 102 to configure or control the pump 122 without needing additional wires or complex physical connection.

In some embodiments, the pump housing 104 may further comprise the slot 300 that defines a J-shaped profile for connecting the gas detector 102 with the pump housing 104. The slot 300 may correspond to a specialized slot or groove formed within the pump housing 104, shaped like the letter "J." The slot 300 may be designed to securely hold or connect the gas detector 102 to the pump housing 104. The slot 300 may further ensure proper mechanical alignment and may provide a way for the first plurality of terminal electrodes to connect correctly with the second plurality of terminal electrodes, when the gas detector 102 is coupled with the pump housing 104.

In some embodiments, the pump 122 may be configured to draw air via a gas input port 202 of the pump housing 104 and transfer the air to a plurality of gas sensors 200 via a plurality of gas output ports 204 to detect the presence of the gas. The pump 122 in the pump housing 104 may draw in the air from an external environment through the gas input port 202. The gas input port 202 on the pump housing 104 may correspond to an entry point for the air in the pump housing 104. In some embodiments, when the gas detector 102 is connected with the pump housing 104, the pump housing 104 may get activated. When the pump housing 104 gets activated, the pump 122 may create a vacuum that may draw the air through the gas input port 202.

In some embodiments, the pump 122 may be configured to move the drawn air from the gas input port 202 through the pump housing 104 and deliver the drawn air to the plurality of gas sensors 200 positioned within the gas detector 102. The plurality of gas sensors 200 may be configured to analyze the delivered air. Once the drawn air reaches the plurality of gas sensors 200, the plurality of gas sensors 200 may detect the presence and concentration of the gas. The detection of the presence and the concentration of the gas may help to monitor the environment for potential hazards.

In some embodiments, the battery management unit 120 may be connected to the first power terminal electrode 106. The battery management unit 120 may be configured to interface with an external power source through the first power terminal electrode 106. The external power source may correspond to a charger. When the gas detector 102 is plugged in or connected to an external power source, the power may flow from the external power source to the first power terminal electrode 106 to reach the battery management unit 120. The battery management unit 120 may be configured to control the charging process by regulating the amount of current sent to the battery 114 based on current charge level and capacity of the battery 114.

In some embodiments, controlled charging process may ensure safe and efficient charging of the battery 114. The controlled charging process may further prevent overcharging or overheating. Further, the battery management unit 120 may track important metrics to assess battery health. The important metrics may include, but is not limited to, voltage, current, and temperature. Further, the battery management unit 120 may be configured to adjust power distribution to ensure optimal performance and longevity of the battery 114. In some embodiments, the battery 114 may be connected to the first plurality of terminal electrodes. The battery 114 may be configured to provide electrical power to different components of the gas detector 102 through the first plurality of terminal electrodes.

In some embodiments, the pump housing 104 may further comprise a contractor board 206. The contractor board 206 may comprise the second plurality of terminal electrodes, the second IR sensor 130, and the plurality of magnets 128. The contractor board 206 may facilitate transmission of control signals between the gas detector 102 and the pump housing 104. In some embodiments, the pump housing 104 may further comprise a sealing ring 208. The sealing ring 208 may provide a secure and airtight connection between the pump housing 104 and gas detector 102, preventing leaks and ensuring proper flow of the gas through the gas input port 202 and the gas output port 204.

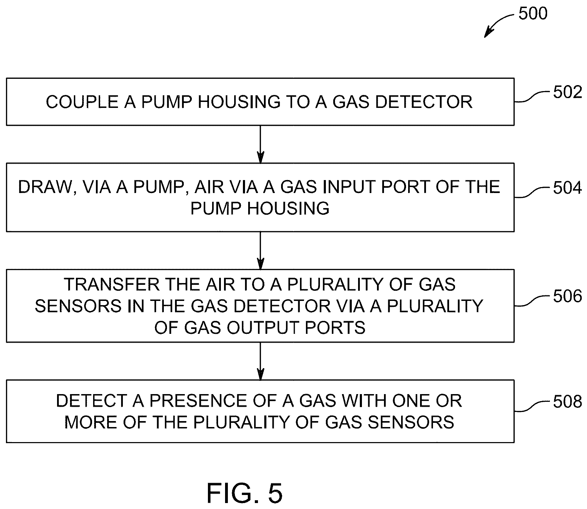

FIG. 5 illustrates a flowchart showing a method 500 for detecting the presence of the gas in accordance with the example embodiment of the present disclosure.

At operation 502, the method 500 may comprise the step of coupling the pump housing 104 to the gas detector 102. Coupling the pump housing 104 to the gas detector 102 may correspond to physically and electrically connecting the pump housing 104 with the gas detector 102. The coupling of the pump housing 104 and the gas detector 102 may be configured to align the first plurality of terminal electrodes to the second plurality of terminal electrodes. Further, the coupling of the pump housing 104 and the gas detector 102 may be configured to align the plurality of switches to the plurality of magnets 128. Further, coupling of the pump housing 104 and the gas detector 102 may allow power transmission from the gas detector 102 to the pump housing 104.

At operation 504, the method 500 may comprise the step of drawing, via the pump 122, the air via the gas input port 202 of the pump housing 104. The pump 122 may be configured to actively pull the air into the pump housing 104 through the gas input port 202 positioned on the pump housing 104.

At operation 506, the method 500 may comprise the step of transferring the air to the plurality of gas sensors 200 in the gas detector 102 via a plurality of gas output ports 204. The air intake process using the gas input port 202 may enable the device 100 to sample the surrounding atmosphere, directing the drawn air towards the plurality of gas sensors 200 that may then detect the presence of the gas. The drawn air may be directed towards the plurality of gas sensors 200 via the plurality of gas output ports 204. Once the pump 122 may draw the air into the pump housing 104 through the gas input port 202, the air may then flow toward the gas detector 102 via the plurality of gas output ports 204. The plurality of gas output ports 204 may correspond to exit points that may transfer the air from the pump housing 104 to the gas detector 102.

At operation 508, the method 500 may comprise the step of detecting the presence of the gas with one or more of the plurality of gas sensors 200. The gas detector 102 may use a variety of the plurality of gas sensors 200 to analyze the air for specific gases, utilizing specialized sensing technologies suited to different types of gases. Each of the plurality of gas sensors 200 may be calibrated to detect particular gas by measuring changes caused when the gases interact with the plurality of gas sensors 200. As the air continuously flows across the plurality of gas sensors 200, the gas detector 102 may interpret the data, and may determine the presence and the concentration of the gas.

The disclosed device 100 offers several advantages, particularly in terms of reliability, energy efficiency, and safety. By utilizing a dual-switch mechanism where the SPDT switch 110 and the SPST switch 112 respond to distinct magnetic field directions, the device 100 ensures precise control over the operation of the pump 122. The device 100 prevents the pump 122 from activating unless the gas detector 102 and the pump housing 104 are correctly coupled, reducing the risk of accidental or unintended operation of the device 100. Additionally, the slot 300 for coupling the gas detector 102 with the pump housing 104 provides a secure and reliable mechanical connection, enhancing the overall stability and functionality of the device 100. The present disclosure integrates the pump 122 directly with the gas detector 102, eliminating the need for a separate power source. This streamlined configuration reduces both equipment cost and complexity for operators. The device's 100 efficient coupling mechanism, utilizing the plurality of switches, enables seamless operation without the need for multiple devices to be managed simultaneously.

Many modifications and other embodiments of the present disclosure set forth herein will come to mind to one skilled in the art to which the present disclosure pertains having the benefit of the teachings presented in the foregoing descriptions and the associated drawings. Therefore, it is to be understood that the present disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A device comprising:
a gas detector comprising:
a first plurality of terminal electrodes;
a plurality of switches; and
a first infrared (IR) sensor; and
a pump housing coupled to the gas detector, the pump housing comprising:
a pump;
a second plurality of terminal electrodes configured to align with the first plurality of terminal electrodes;
a plurality of magnets configured to control the plurality of switches to enable or disable flow of electricity from the first plurality of terminal electrodes to the second plurality of terminal electrodes to operate the pump for detecting a presence of a gas; and
a second infrared (IR) sensor configured to communicate with the first IR sensor of the gas detector.

2. The device of claim 1, wherein the pump is configured to draw air via a gas input port of the pump housing and transfer the air to a plurality of gas sensors via a plurality of gas output ports to detect the presence of the gas.

3. The device of claim 1, wherein the pump housing further comprises a slot that defines a J-shaped profile for connecting the gas detector with the pump housing.

4. The device of claim 1, wherein the first plurality of terminal electrodes comprises a first power terminal electrode and a first ground terminal electrode and the second plurality of terminal electrodes comprise a second power terminal electrode and a second ground terminal electrode.

5. The device of claim 4, wherein the gas detector further comprises a battery management unit connected to the first power terminal electrode and a battery connected to the first plurality of terminal electrodes, wherein the pump housing further comprises a power management unit and a plurality of gas output ports.

6. The device of claim 5, wherein the plurality of switches comprises a single pole double throw (SPDT) switch connected to the first power terminal electrode and a single pole single throw (SPST) switch connected to the battery.

7. The device of claim 6, wherein the SPDT switch and the SPST switch are configured to respond to a magnetic field of the plurality of magnets such that:
when the pump housing is coupled to the gas detector, the SPDT switch is in an open state and the SPST switch is in a closed state; and
when the pump housing is not coupled to the gas detector, the SPDT switch is in a closed state and the SPST switch is in an open state.

8. The device of claim 7, wherein when the SPDT switch is in the open state and the SPST switch is in the closed state, the battery is configured to transmit power to the pump via the first power terminal electrode and the second power terminal electrode.

9. The device of claim 7, wherein when the SPDT switch is in the closed state and the SPST switch is in the open state, the battery management unit is configured to control supply of power received from an external power source to recharge the battery.

10. The device of claim 6, wherein the SPST switch is positioned at a different direction with the SPDT switch so that the SPST switch is configured to be sensitive to a different magnetic field direction than the SPDT switch.

11. A method comprising:
coupling a pump housing to a gas detector, wherein the gas detector comprises:
a first plurality of terminal electrodes;
a plurality of switches; and
a first infrared (IR) sensor; and
wherein the pump housing comprises:
a pump;
a second plurality of terminal electrodes configured to align with the first plurality of terminal electrodes;
a plurality of magnets configured to control the plurality of switches to enable or disable flow of electricity from the first plurality of terminal electrodes to the second plurality of terminal electrodes to operate the pump for detecting a presence of a gas; and
a second infrared (IR) sensor configured to communicate with the first IR sensor of the gas detector.

12. The method of claim 11 further comprising:
drawing, via the pump, air via a gas input port of the pump housing;
transferring the air to a plurality of gas sensors in the gas detector via a plurality of gas output ports; and
detecting the presence of the gas with one or more of the plurality of gas sensors.

13. The method of claim 11, wherein the pump housing further comprises a slot that defines a J-shaped profile for connecting the gas detector with the pump housing.

14. The method of claim 11, wherein the first plurality of terminal electrodes comprises a first power terminal electrode and a first ground terminal electrode and the second plurality of terminal electrodes comprise a second power terminal electrode and a second ground terminal electrode.

15. The method of claim 14, wherein the gas detector further comprises a battery management unit connected to the first power terminal electrode and a battery connected to the first plurality of terminal electrodes, wherein the pump housing further comprises a power management unit and a plurality of gas output ports.
